Europäisches Patentamt

European Patent Office

Office européen des brevets

(19)

(11) Veröffentlichungsnummer : **0 365 668 B1**

(12) # EUROPÄISCHE PATENTSCHRIFT

(45) Veröffentlichungstag der Patentschrift :
30.10.91 Patentblatt 91/44

(51) Int. Cl.⁵ : **B01J 19/24, // C07H21/00**

(21) Anmeldenummer : **89906098.2**

(22) Anmeldetag : **24.04.89**

(86) Internationale Anmeldenummer :
**PCT/EP89/00447**

(87) Internationale Veröffentlichungsnummer :
**WO 89/10188 02.11.89 Gazette 89/26**

(54) **VORRICHTUNG ZUR DURCHFÜHRUNG CHEMISCHER REAKTIONSFOLGEN.**

(30) Priorität : **22.04.88 DE 3813671**

(43) Veröffentlichungstag der Anmeldung :
**02.05.90 Patentblatt 90/18**

(45) Bekanntmachung des Hinweises auf die
Patenterteilung :
**30.10.91 Patentblatt 91/44**

(84) Benannte Vertragsstaaten :
**AT BE CH DE FR GB IT LI LU NL SE**

(56) Entgegenhaltungen :
**EP-A- 0 164 206**
**EP-A- 0 181 491**

(73) Patentinhaber : **EUROPÄISCHES**
**LABORATORIUM FÜR MOLEKULARBIOLOGIE**
**(EMBL)**
**Meyerhofstrasse 1**
**W-6900 Heidelberg 1 (DE)**

(72) Erfinder : **ANSORGE, Wilhelm**
**Heidelbergstrasse 49**
**W-6901 Gaiberg über Heidelberg (DE)**
Erfinder : **SCHWAGER, Christian**
**Albert-Schweitzer-Strasse 4a**
**W-6703 Limburgerhof (DE)**
Erfinder : **SPROAT, Brian**
**Röntgenstrasse 38**
**W-6900 Heidelberg (DE)**

(74) Vertreter : **Prechtel, Jörg et al**
**Patentanwälte H. Weickmann, Dr. K. Fincke**
**F.A. Weickmann, B. Huber Dr. H. Liska, Dr. J.**
**Prechtel Möhlstrasse 22 Postfach 860 820**
**W-8000 München 86 (DE)**

## Beschreibung

Die Erfindung betrifft eine Vorrichtung zur Durchführung chemischer Reaktionsfolgen mit

- einem Stapel übereinander angeordneter und wahlweise gegeneinander in zur Stapelrichtung senkrechter Verschieberichtung schrittweise verschiebbarer Reaktionsplatten, die mit im Schrittabstand angeordneten Durchgängen versehen sind, von denen jeweils eine als Reaktionskammer ausgebildet ist,
- einer Plattenverstelleinrichtung zur wahlweisen Verschiebung der jeweiligen Platte relativ zum übrigen Plattenstapel mittels eines wahlweise antreibbaren Plattenverschiebeteils. welches an einem in Stapelrichtung zwischen den einzelnen Platten zugeordneten Plattenverstellpositionen wahlweise bewegbaren Kopf der Plattenverstelleinrichtung beweglich gelagert ist, und
- einer Spanneinrichtung mit einem an den Plattenstapel angreifenden Andrückteil, welches zwischen einer Andrückstellung mit abdichtendem Zusammenspannen der Platten und einer Freigabestellung mit Bewegungsmöglichkeit der Platten gegeneinander bewegbar ist.

Eine Vorrichtung dieser Art ist bekannt (EP-A-164 206). Mit ihr lassen sich gleichzeitig eine Reihe von biochemischer Reaktionen durchführen, insbesondere die Synthese von DNA-Bruchstücken, wie dies auch aus der EP-A-181 491 an sich unter Verwendung eines Stapels gegeneinander manuell zu verdrehender kreisscheibenförmiger Platten bekannt ist. Über vier Durchgänge werden die Basen A, C, G, T dem Stapel zugeführt. Entsprechend der gewünschten Sequenz wird die Reaktionskammer der jeweiligen Platte in Flucht mit dem die gewünschte Base liefernden Durchgang gebracht. Die aus der eingangs genannten EP-A-164 206 bekannte Vorrichtung erlaubt ein automatisches Verschieben (Verdrehen) der einzelnen Platten gegeneinander, so daß eine der Plattenanzahl entsprechende Vielzahl gleichzeitiger Synthesereaktionen mit hoher Reaktionsschritt-Anzahl rechnergestützt ohne manuellen Eingriff durchgeführt werden kann. Der apparative Aufwand ist jedoch bei dieser bekannten Lösung beträchtlich. Es sind insgesamt vier Antriebe erforderlich: ein Höhenantrieb 70, der eine Anstriebsspindel 64 für die Höhenverstellung der Plattenverschiebeeinrichtung sowie eine Antriebsspindel 63 für eine Verschiebesperre antreibt; ein Antrieb 75 als Teil der Plattenverschiebeeinrichtung, der ein Plattenverschiebeteil in Form einer über einen weiteren Antrieb 77 wahlweisen antreibbaren Andruckrolle 76 in und außer Eingriff mit dem Außenumfang der jeweiligen Platte bringt; ein vierter Antrieb in Form eines Hub-Zylinders 62 der der Bewegung des Andrückteils in Form einer Platte dient. Diese Platte ist oberhalb des Plattenstapels angeordnet und zwischen der Freigabestellung, in welcher die Platten zur Vorbereitung der nächsten Reaktion gegeneinander verdrehbar sind, und der Andrückstellung, in welcher die Platten vom Andrückteil dichtend zusammengespannt werden, bewegbar. In der Andrückstellung wird der jeweilige Reaktionsschritt durchgeführt.

Die Aufgabe der Erfindung liegt demgegenüber darin, eine Vorrichtung der eingangs genannten Art mit vereinfachtem Aufbau bei zuverlässiger Funktion bereitzustellen.

Diese Aufgabe wird dadurch gelöst, daß die Spanneinrichtung eine mit dem Andrückteil verkoppelte Kraftübertragungseinrichtung umfaßt, an welche die Plattenverstelleinrichtung angreift zur Bewegung des Andrückteils aus der Freigabestellung in die Andrückstellung bei einer Bewegung des Kopfes der Plattenverstelleinrichtung über eine der beiden äußeren Plattenverstellpositionen hinaus in eine Endposition.

Erfindungsgemäß wird also das Andrückteil von der Plattenverstelleirnichtung betätigt, so daß ein eigener Antrieb für das Andrückteil entfallen kann.

Eine besonders einfache erfindungsgemäße Bauform ist dadurch gekennzeichnet, daß die Kraftübertragungseinrichtung einen Doppelarmhebel umfaßt, desse eines Ende mit der Plattenverstelleinrichtung und dessen anderes Ende mit dem Andrückteil zusammenwirkt. Der Doppelarmhebel überträgt also die Aufwärtsbewegung der Plattenverstelleinrichtung mit Vorzeichenumkehr auf das nach unten zu bewegende Andrückteil.

Zur genauen Spanndruckeinstellung, beispielsweise nach Änderung der Reaktionsplattenanzahl, wird vorgeschlagen, daß wenigstens eines der Enden des Doppelarmhebels mit einer Spanndruck-Einstelleinrichtung versehen ist. Bevorzugt ist hierbei vorgesehen, daß die Spanndruck-Einstelleinrichtung von einem verstellbare Doppelkeil gebildet ist.

Bei der eingangs genannten bekannten Vorrichtung ist eine in Stapelrichtung mit der Plattenverstelleinrichtung bewegbare Verschiebesperre (in Form der Gewindespindel 63) vorgesehen, welche diejenige Platte unmittelbar oberhalb der jeweils von der Plattenverstelleinrichtung angefahrenen Platte blockiert, indem diese entsprechend weit von oben her in miteinander fluchtende Durchgangsbohrungen der Platten eingeschoben ist. Jede Platte ist mit einer Reihe entsprechend dem Schrittabstand voneinander beabstandeten Durchgangsbohrungen dieser Art versehen. Nachteilig an dieser Anordnung ist, daß die unterhalb der zu verstellenden Platte angeordnete Platte nicht fixiert ist, so daß es zu einem unkontrollierte Mitdrehen dieser Platte kommen kann. Wird dieses Mitdrehen nicht korrigiert, so wird bei der nächsten Reaktion möglicherweise in dieser Platte eine falsche Reaktion durchgeführt. Noch ungünstiger wäre ein nur teilweises Mitdrehen dieser Platte, da dann möglicherweise sämtliche Durchgänge durch diese Platte gesperrt sind.

Alternativ oder zusätzlich zu den vorstehend beschriebenen erfindungsgemäßen Maßnahmen wird zur Erhöhung der Funktionssicherheit der Vorrichtung der eingangs genannten Art bei einfachem Aufbau vorgeschlagen, daß die Verschiebesperre auch an die unmittelbar unterhalb der von der Plattenverstelleinrichtung angefahrenen Platte sperrend angreift.

Zur Vereinfachung des mechanischen Aufbaus, insbesondere durch Wegfall des Spindelantriebs, bei der bekannten Vorrichtung, wird vorgeschlagen, daß am Kopf oberhalb und unterhalb des Plattenverschiebeteils je ein Eingriffselement starr angeordnet ist, welches mit an den Platten angeordneten Gegeneingriffselementen zusammenwirkt.

Um wiederum in baulich einfache Weise eine zuverlässige Fixierung der jeweiligen Platten in ihren sämtlichen möglichen Verschiebepositionen sicherzustellen, wird vorgeschlagen, daß eines der Elemente Eingriffselement - Gegeneingriffselement einen Eingriffsvorsprung umfaßt, und daß das andere Element mehrere, in Verschieberichtung nebeneinander im Schrittabstand angeordnete, zur Stapelrichtung parallel verlaufende, im Querschnitt an den Eingriffsvorsprung angepaßte Eingriffs-Nuten umfaßt.

Alternativ kann jedoch auch vorgesehen sein, daß eines der Elemente Eingriffselement - Gegeneingriffselement eine zur Stapelrichtung parallel verlaufende Eingriffs-Nut umfaßt, und daß das andere Element mehrere, in Verschieberichtung nebeneinander im Schrittabstand angeordnete, an den Nut-Querschnitt angepaßte Eingriffsvorsprünge umfaßt.

Bei Ausbildung der erfindungsgemäßen Vorrichtung mit einem am oberen Stapelende angeordneten Andrückteil wird vorgeschlagen, diese mit einer Eingriffs-Nut für den oberen Eingriffsvorsprung des Kopfes auszubilden.

Gemäß einer weiteren Ausbildung der Erfindung, wird vorgeschlagen, daß das Plattenverschiebeteil von einem in Verschieberichtung beweglichen Verschiebevorsprung gebildet ist, welcher mit an den Platten ausgebildeten Gegenvorsprüngen zusammenwirkt. Besonders bevorzugt ist hierbei vorgesehen, daß der Verschiebevorsprung in einer Zwischenposition der Plattenverschiebeeinrichtung zwischen aufeinanderfolgenden Plattenverstellpositionen frei zwischen den Gegenvorsprüngen aufeinanderfolgender Platten hindurch bewegbar ist. Dies hat den großen Vorteil, daß der bei der bekannten Vorrichtung erforderliche Antrieb 75 zur Horizontalbewegung des Kopfes in und außer Eingriff mit dem Plattenstapel entfallen kann.

Der Einfachheit halber kann hierbei vorgesehen sein, daß der Verschiebevorsprung und/oder die Gegenvorsprünge stiftförmig sind.

Im Normalbetrieb bei einer mittleren Anzahl von Platten, beispielsweise 10 Platten, muß nicht befürchtet werden, daß die eine oder andere Platte bei der Verschiebung klemmt. Sollte ein derartiges Klemmen dennoch auftreten, was vor allem bei erhöhter Plattenanzahl vorkommen kann, wird vorgeschlagen, daß die Plattenverschiebeeinrichtung mit einer Überlastsicherung versehen ist.

Eine derartige, sich durch einfachen Aufbau und zuverlässige Funktion auszeichnende Überlastsicherung ist erfindungsgemäß dadurch gekennzeichnet, daß das stiftförmige Plattenverschiebeteil elektrisch leitfähig ausgebildet ist, daß eine Vorspannfeder in einer Normalstellung des Plattenverschiebeteils das Plattenverschiebeteil gegen ein Isolierteil andrückt, und daß bei Überlast das Plattenverschiebeteil vom Isolierteil abgleitet und von der Vorspannfeder gegen eine elektrisch leitende Kontaktplatte gedrückt wird.

Um auch bei relativ geringem Zusammenspann-Druck eine zuverlässige Dichtung der Durchgänge im Übergangsbereich aufeinanderfolgender Platten zu sichern, werden O-Ring-Dichtungen zwischen aufeinanderfolgenden Platten vorgeschlagen.

Die Erfindung wird im folgenden an einem bevorzugten Ausführungsbeispiel an Hand der Zeichnung erläutert. Es zeigt:

Fig. 1 Eine vereinfachte isometrische Gesamtansicht der erfindungsgemäßen Vorrichtung;

Fig. 2 eine Seitenansicht der Anordnung in Fig. 1 (Blickrichtung II);

Fig. 3 einen Schnitt nach Linie III-III in Fig. 2;

Fig. 4 eine explosionsartige Darstellung der Reaktionsplatten der Vorrichtung gemäß Figuren 1 bis 3;

Fig. 5 einen Schnitt nach Linie V-V in Fig. 4;

Fig. 6 einen Schnitt nach Linie VI-VI in Fig. 4 und

Fig. 7 eine Detailansicht einer abgewandelten Ausführungsform der Erfindung im Bereich des stiftförmigen Plattenverschiebeteils mit Überlastsicherung.

Die erfindungsgemäße, in den Figuren mit 10 bezeichnete Vorrichtung dient der gleichzeitigen Durchführung mehrerer chemischer Reaktionsfolgen. Besonders bevorzugt ist die Anwendung der Vorrichtung im Bereich biochemischer Reaktionsketten, insbesondere der gleichzeitigen Synthese mehrerer, auch unterschiedlich aufgebauter DNA-Stränge. Die Vorrichtung 10 weist einen Stapel 12 aneinander anliegender Reaktionsplatten 14 auf, die im dargestellten Ausführungsbeispiel vertikal übereinander angeordnet sind. Prinzipiell ist jedoch auch eine andere Stapelrichtung, beispielsweise in einer Horizontalebene, denkbar. Jeder Reaktionskette (DNA-Strang) ist jeweils eine Platte 14 zugeordnet.

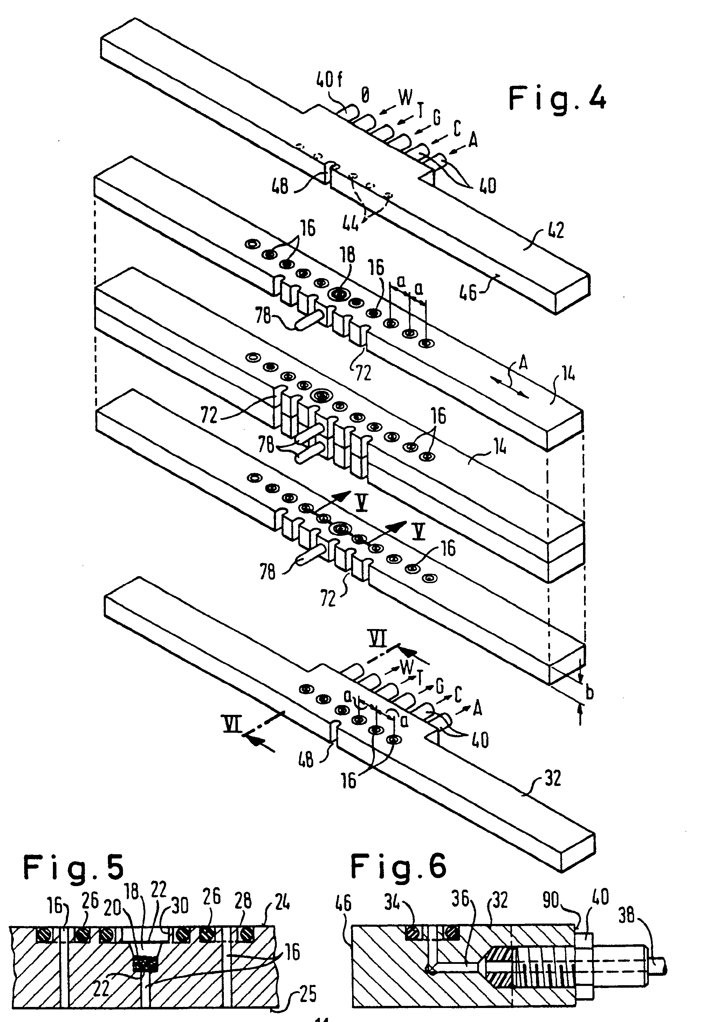

Zur Durchführung der gewünschten Reaktion ist die jeweilige Platte mit einer Reihe von Durchgängen 16 versehen, von denen einer mit einer als Reaktionskammer 18 dienenden Erweiterung ausgebildet ist, wie die Figuren 4 und 5 zeigen. Die Reaktionskammer 18 kann mit Anlagekörpern 20, beispielsweise in Form von kleinen Glaskügelchen (englisch: beads), gefüllt sein, wobei eine Siebplatte 22 am unteren und bevorzugt auch am oberen Ende der Reaktionskammer 18 ein Entweichen der Anlagekörper aus der Reaktionskammer 18 ausschließen.

An der jeweiligen Plattenoberseite 24 befinden sich O-Ring-Dichtungen in Form von O-Ringen 26, die in Ring-nuten 28 eingesetzt sind. Diese Ring-Nuten 28 dienen der Abdichtung der Durchgänge 16 radial nach außen hin bei Anlage der Plattenoberseite 24 an der Plattenunterseite 25 der nächstfolgenden Platte. Die Ring-Nuten 28 umringen daher die Durchgänge 16 konzentrisch mit geringem Abstand; eine der Ring-Nuten hat größeren Durchmesser, nämlich die der Abdichtung der Reaktionskammer 18 dienende Ring-Nut, da die Reaktionskammer 18 nach oben hin in eine Erweiterung 30 übergeht, die in die Plattenoberseite 24 ausmündet

Dementsprechend ist auch eine sich an den Plattenstapel 12 nach unten hin anschließende Anschlußplatte 32 an ihrer Oberseite mit O-Ring-Dichtungen (O-Ring 34 in Fig. 6) versehen, die der Abdichtung jeweils eines von insgesamt sechs Durchgängen 36 dienen. Während die Durchgänge 16 vertikal verlaufen, sind die Kanäle 36 abgeknickt, um einen horizontalen Anschluß von Ableitungen 38 zu ermöglichen. In Fig. 6 erkennt man ein horizontal in die Anschlußplatte 32 eingeschraubtes Leitungsanschlußstück 40.

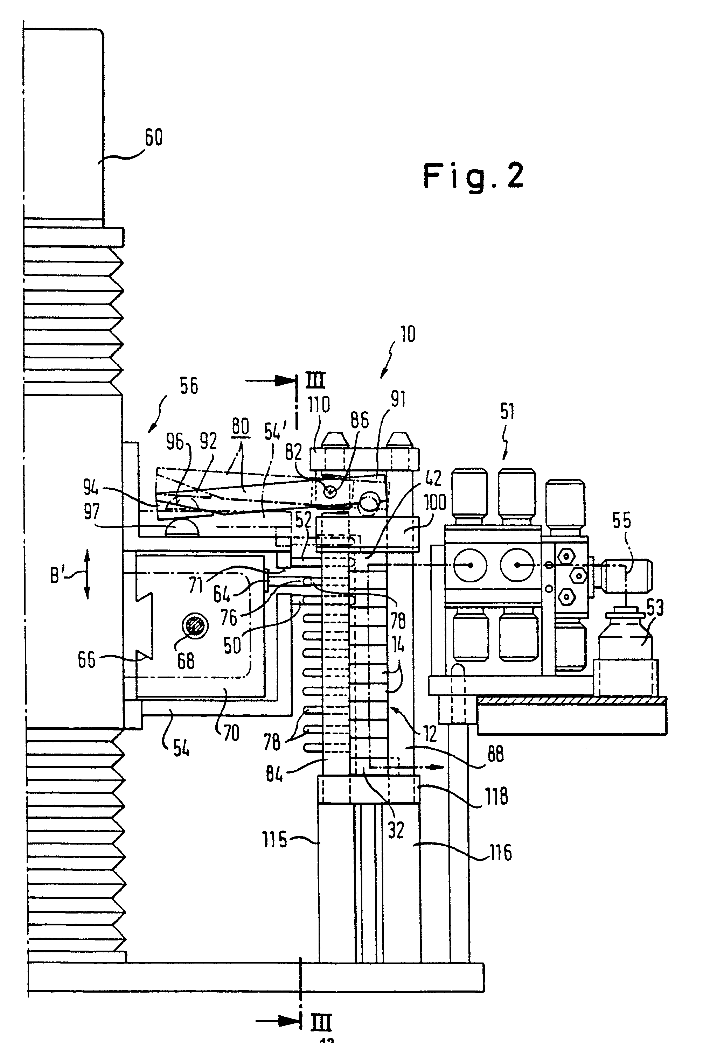

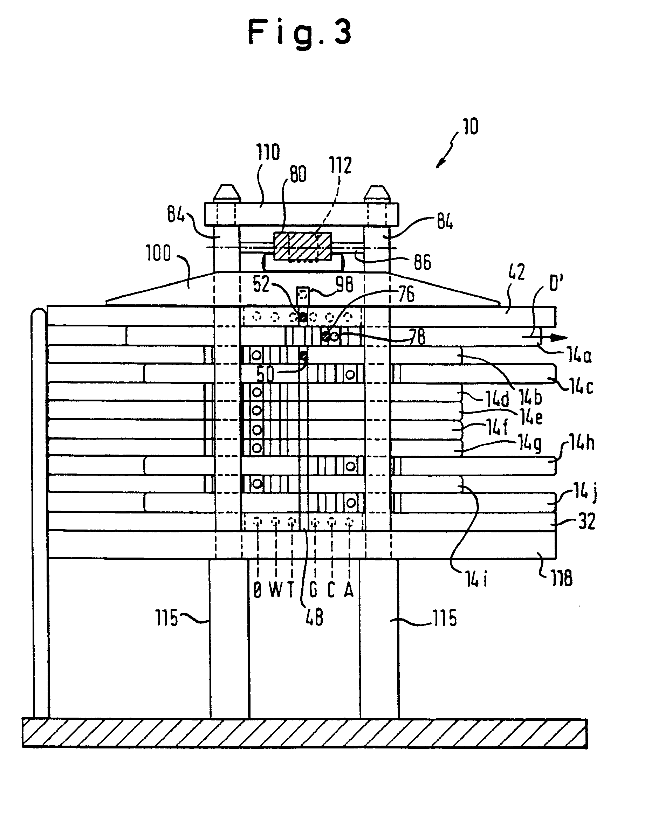

Der unteren Anschlußplatte 32 entspricht eine obere Anschlußplatte 42, wiederum mit sechs horizontalen Anschlußstücken 40, an die sechs abgeknickte Durchgänge angeschlossen sind mit in Fig. 4 strichliert angedeuteten Auslaß-Mündungen 44 an der Plattenunterseite. Beide Anschlußteile 32 und 42 sind in ihrer Längenmitte an der den Anschlußstücken 40 gegenüberliegenden vertikalen Längsseite 46 mit einer vertikal verlaufenden Nut 48 versehen, in die ein unterer bzw. oberer stiftförmiger Eingriffsvorsprung 50 bzw. 52 eindringen kann. Diese Vorsprünge 50 und 52 sind an einem höhenverschiebbaren Kopf 54 einer Plattenverstelleinrichtung 56 fest angebracht (s. Figuren 2 und 3). Zu den Durchgängen 16 und 36 ist noch zu ergänzen, daß diese gleichabständig und in gleicher Breitenlage jeweils auf einer Linie an den Platten 14 bzw. 32 und 42 angeordnet sind, so daß bei entsprechender gegenseitiger Ausrichtung der Platten 32,14 und 42 die Durchgänge in gegenseitige Fluchtung gebracht werden können. Bei einer Verschiebung der einen oder anderen Platte 14 gegenüber den übrigen Platten in Plattenlängsrichtung A um eine Strecke a, die dem Achsabstand unmittelbar aufeinanderfolgender Durchgänge 16 entspricht, so ergibt sich aufgrund dieses einheitlichen Durchgangsrasters wiederum eine fluchtende Anordnung eines Teils der Durchgänge der soeben verschobenen Platte mit Durchgängen der auf diese Platte nach oben und nach unten folgenden Platten 14,32 und 42.

Die Platten 32 und 42 sind entsprechend der Anzahl der unterschiedlichen Reaktionsflüssigkeiten mit Durchgängen 36 versehen. Im vorliegenden Beispiel werden vier Anschlüsse benötigt für die vier Basen A, G, C und T. Ein weiterer Kanal dient der Zuführung von Hilfsflüssigkeiten, wie z.B. Waschflüssigkeit. In Fig. 4 sind dementsprechend Zuführungspfeile mit A, C, G, T und W eingezeichnet. Ein äußerer Kanal (Pfeil O) kann unbeaufschlagt bleiben und definiert dann eine Parkstellung für die Reaktionskammern 18 der Platten 14. Es ergeben sich insgesamt n Anschlüsse (n = 6) mit dementsprechend sechs Anschlußteilen 40 in der Platte 42 und in der Platte 32. Bei Bedarf können auch noch weitere Anschlüsse vorgesehen sein.

Demgegenüber sind die Platten 14 mit jeweils insgesamt m Durchgängen 16 (m=2n-1=11) versehen, wobei der mittlere Durchgang zur Durchgangskammer 18 erweitert ist. Die jeweilige Reaktionskammer 18 kann demzufolge wahlweise in Flucht mit jedem beliebigen der sechs Anschlüsse der Platten 32 und 42 gebracht werden, wobei stets auch jeweils einer der übrigen Durchgänge 16 in Flucht mit sämtlichen übrigen Anschlüssen liegt. Demzufolge kann stets durch die sechs Anschlußteile 40 der oberen Platte 42 entsprechende Flüssigkeit zugeführt, durch die Durchgänge 16 der Platten 14 des Plattenstapels 12 zur unteren Platte 32 geführt und durch die unteren Anschlußteile 40 abgeleitet werden.

Die Reaktionskammer 18 einer beliebigen Platte 14 kann wahlweise in Kontakt mit einer der Flüssigkeiten A, C, G, T oder W gebracht werden, oder in eine Parkstellung in Flucht mit dem in Fig. 4 am weitesten links gelegenen Anschluß (Anschlußteil 40f).

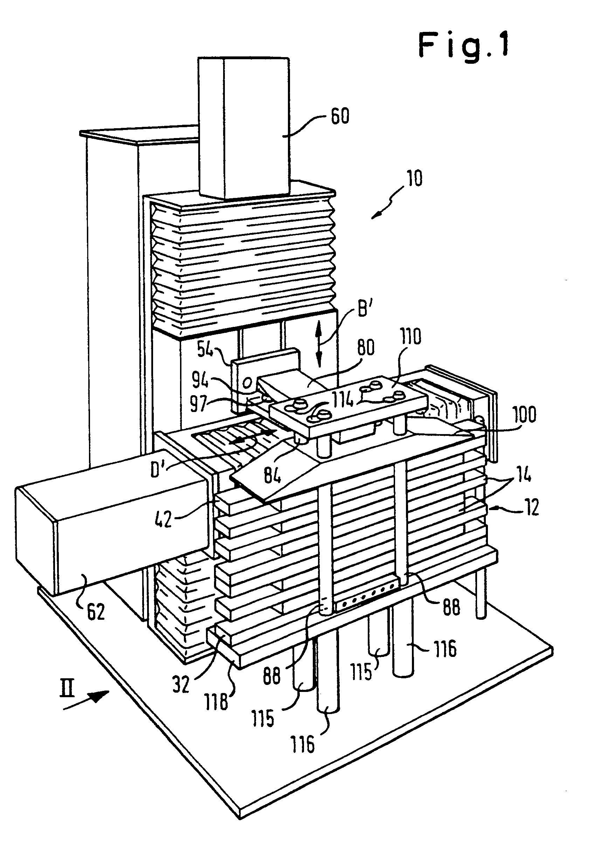

In Fig. 1 sind die Zuleitungen zur Platte 42 und die Ableitungen 38 weggelassen ebenso wie ein zugehöriger Ventilblock mit Vorratsflaschen. In Fig. 2 ist dagegen der Ventilblock schematisch dargestellt und mit 51 bezeichnet. Ferner erkennt man eine Vorratsflasche 53 der wenigstens fünf Vorratsflaschen. Mit einer Strich-Punkt-Linie 55 ist der Flüssigkeitsfluß aus der Vorratsflasche 53 in den Ventilblock 51 und weiter in die obere Platte 42, dann durch den Plattenstapel 12 hindurch nach unten zur unteren Platte 32 angedeutet. Anschließend wird die Flüssigkeit entweder rückgeführt oder einem Sammelbehälter zugeführt.

Der Ventilblock 51 kann auch mit 2/1-Wegeventil ausgebildet sein, wobei dem einen der beiden Eingänge eine erste Flüssigkeit und dem anderen der beiden Eingänge eine zweite Flüssigkeit zugeführt wird. Durch den einen Ausgang kann nun wahlweise entweder nur die eine oder nur die andere oder ein Gemisch aus beiden Flüssigkeiten mit vorgegebenem Mischungsverhältnis zugeführt werden, wobei in letzterem Falle das Ventil in kurzen Zeitabständen umgeschalte wird (nach Art eines Flip-Flop-Elements). Das Mischungsverhältnis wird dann durch das Verhältnis der Verweildauer des Ventils in den beiden Schaltstellungen definiert. Dieses 2/1-Wegeventil läßt natürlich auch durch zwei einfache Schaltventile realisieren, die in eine gemeinsame Ableitung münden und die wechselweise auf Durchlaß geschaltet werden, so daß entweder die eine oder die andere Flüssigkeit der gemeinsamen Ableitung zugeführt wird.

Um den gewünschten Flüssigkeitsdruck zu erhalten, damit die Flüssigkeiten durch den Stapel 12 fließen, kann in üblicher Weise eine Druckbeaufschlagung der Flaschen 53 mit Druckgas erfolgen. Es können auch Flüssigkeitspumpen, wie z.B. Dosier-Spritzen, für exakte Volumenzumessung eingesetzt werden.

Die Plattenverstelleinrichtung 46 umfaßt den bereits erwähnten Kopf 54, der in vertikaler Richtung B' mit Hilfe eines nicht näher dargestellten Antriebs mit oben angeordnetem Antriebsmotor 60 hin und her bewegbar ist. Der Kopf 54 wiederum trägt einen nicht näher dargestellten Horizontalantrieb mit seitlichem Antriebsmotor 62 zur wahlweisen Horizontalverschiebung (Pfeil D' in Fig. 1) eines stiftförmigen Plattenverschiebeteils 64. In Fig. 2 ist eine Schwalbenschwanzführung 66 zwischen kopf 54 und einem das Plattenverschiebeteil 64 tragenden und über eine Gewindespindel 68 relativ zum Kopf 54 hin und her verschiebbaren Führungsteil 70 angedeutet. Im Bereich des Plattenverschiebeteils 64 ist der das Teil 70 gehäuseartig umschließende Kopf 54 mit einem Durchtrittsschlitz 71 versehen. Das Plattenverschiebeteil 64 ragt in horizontaler Richtung aus dem Schlitz 71 vor und verläuft somit parallel und im gleichen Vertikalabstand zu den beiden stiftförmigen Eingriffsvorsprüngen 50 und 52. Der Abstand zwischen dem stiftförmigen Plattenverschiebeteil 64 und den Eingriffsvorsprüngen 50 und 52 entspricht jeweils der Plattendicke b der Platten 14, 32 und 42, so daß dann, wenn das Plattenverschiebeteil 64 einer Platte 14 gegenübersteht, die beiden Eingriffsvorsprünge 50 und 52 der nächstfolgenden unteren bzw. oberen Platte 14 bzw. 32 bzw. 42 gegenüberliegen.

Die beiden stiftförmigen Eingriffsvorsprünge 50 und 52 dienen der momentanen Fixierung der Platten 14 beidseits der momentan durch das stiftförmige Plattenverschiebeteil 64 zu verstellenden Platte 14, um die Reaktionskammer 18 dieser Platte in den Strom einer anderen der zur Verfügung stehenden Flüssigkeiten zu bringen oder in Parkstellung.

Hierzu sind sämtliche Platten 14 mit den Nuten 48 entsprechenden Nuten 72 versehen. Da jede Platte 14, wie bereits erläutert, insgesamt sechs Verschiebestellungen im Raster der Lochabstände a einnehmen kann, ist demzufolge jede Platte 14 mit insgesamt sechs dieser Nuten 42 mit gegenseitigem Nutabstand a ausgebildet. In jeder der sechs Verschiebepositionen fluchtet eine dieser Nuten 72 mit den beiden Nuten 48 der unteren und oberen Platte 32 und 42. Befinden sich sämtliche Platten 14 in einer ihrer Verschiebepositionen, so kann der Kopf 54 ungehindert nach oben oder unten verfahren werden, wobei die beiden freien Enden der stiftförmigen Eingriffsvorsprünge 50 und 52 innerhalb dieser aus den miteinander fluchtenden Nuten 48,72 gebildeten Gesamt-Nut nach oben oder unten bewegt werden.

Zur Verstellung einer der Platten 14 wird der Kopf 54 in die entsprechende Höhenposition gefahren, so daß das stiftförmige Plattenverschiebeteil 64 der gewünschten Platte 14 gegenüberliegt. Die beiden Eingriffselemente 50 und 52 greifen dann blockierend in die nut 72 der beiden Platten beidseits der zu verstellenden Platte ein. Zur Bewegung dieser Platte wird das Plattenverschiebeteil mit seinem als Verschiebevorsprung 76 dienenden freien Ende zur seitlichen Anlage an einen stiftförmigen Gegenvorsprung 78 gebracht, welcher letzterer von der gegenüberliegenden Platte 14 horizontal absteht. Sodann erfolgt die gewünschte Seitwärtsverschiebung der Platte 14 um den ein- oder mehrfachen Abstand a.

Um dieselbe Platte 14 auch in Gegenrichtung verschieben zu können, wird der Verschiebevorsprung 76 in einer Zwischenstellung des Kopfes 54 in Höhe der Berührungsfläche aufeinanderfolgender Platten ober- oder unterhalb des Gegenvorsprungs 76 an diesem vorbeibewegt. Anschließend fährt der Kopf 54 in die gewünschte Höhenposition, so daß dann der Verschiebevorsprung 78 den Gegenvorsprung 76 in der gewünschten Gegenrichtung verschieben kann.

Damit der Verschiebevorsprung 76 zwischen den Gegenvorsprüngen 78 aufeinanderfolgender Platten in horizontaler Richtung hindurch bewegt werden kann (in der besagten Zwischenposition des Kopfes 54), ist der Stiftdurchmesser der Gegenvorsprünge 78 sowie des Verschiebevorsprungs 76 kleiner als die halbe Plattendicke b.

Um nach erfolgter Plattenverschiebung bei der anschließenden Beaufschlagung der sechs durchgehenden Kanäle eine einwandfreie Dichtung dieser Kanäle nach außen hin sicherzustellen, also eine ausreichende Abdichtung durch die O-Ringe 26,34 der O-Ring-Dichtungen zwischen sämtlichen Platten (14,32 und 42), werden diese Platten mit Hilfe eines Doppelarmhebels 80 in Stapelrichtung (vertikale Richtung B') zusammengespannt. Dieser Doppelarmhebel ist über eine Lagerwelle 82 an zwei vorderen Säulen 84 mit horizontaler Schwenkachse 86 drehbar gelagert. Die beiden Säulen 84 bilden eine vordere Führung für die Platten 14 mit Anlage der Plattenvorderseiten 46 an den Säulen 84. Hintere Säulen 88 liegen dementsprechend an den Plattenrückseiten 90 an. Um bei relativ geringer Betätigungskraft eine hohe Vorspannkraft zu erhalten, ist der an den Plattenstapel andrückende Arm 91 des Doppelarmhebels 80 mit wesentlich geringerer effektiver Länge ausgebildet wie der andere, vom Kopf 54 zu betätigende Arm 92.

Zur genauen Hebeljustierung und damit auch zur Einstellung der gewünschten Vorspannkraft, ist der Arm 80 mit einer Art Doppelkeil ausgebildet. Ein Teil 94 ist längs einer Keilfläche 96 des Arms 92 verschiebbar und in der gewünschten Position fixierbar. Am Keil 94 schlägt ein am Kopf 54 befestigtes halbkugelförmiges Teil 97 an, sobald der Kopf 54 über die oberste Plattenverstellposition hinaus nach oben verschoben wird. In der obersten Plattenverstellposition liegt der Verschiebevorsprung 76 in gleicher Höhe wie der Gegenvorsprung 78 der obersten Platte 14. In dieser Stellung befindet sich der obere Eingriffsvorsprung 52 in der Nut der Platte 42. Bei der anschließenden Bewegung des Kopfes 54 weiter nach oben in eine obere Endposition (in Fig. 2 mit einer Strich-Punkt-Linie angedeutet und mit 54' bezeichnet) dringt der obere Eingriffsvorsprung 52 in eine dementsprechend vertikal fluchtend angeordnete Nut 98 eines auf der Platte 42 aufliegenden Andrückteils 100 ein. Gleichzeitig bewegt sich das Andrückteil 100 unter der Wirkung des Doppelarmhebels 80 geringfügig nach unten, so daß der Plattenstapel 12 dementsprechend zusammengedrückt wird. Die vertikale Nutlänge der Nut 98 ist dementsprechend groß.

Nach erfolgter Reaktion zur nachfolgenden Plattenverstellung wird der Kopf 54 wieder nach unten gefahren, so daß der Doppelarmhebel 80 frei wird und die Platten 14 wieder lose aufeinander liegen. Sie können also wieder seitlich verschoben werden.

In der Anordnung gemäß Fig. 3 wird momentan die oberste Platte 14a in ihre am weitesten rechts gelegene Stellung verschoben (Pfeil D'), so daß dann deren Reaktionskammer in der nächstfolgenden Reaktion von einer Flüssigkeit mit der Base A durchsetzt wird. Die nächstfolgende Platte 14b ist in Parkstellung; die nächstfolgende Platte 14c ist wiederum in der der Base A zugeordneten Stellung; die nächstfolgenden Platten 14c bis 14g sind in Parkposition, ebenso wie die Platte i. Die Platten 14h und 14j befinden sich in der der Base A zugeordneten Position.

Zur Lagerung des Doppelarmhebels 80 sei noch erwähnt, daß es auch alternativ möglich ist, diesen an einer von allen vier Säulen 84 und 88 durchsetzten Kopfplatte 110 im Bereich der oberen Säulenenden drehzulagern; ein in den Doppelarmhebel 80 eingreifender und an der Platte 110 starr befestigter Lagerbock ist in Fig. 3 mit punktierter Umrißlinie angedeutet und mit 112 bezeichnet. Wie ferner aus Fig. 1 zu entnehmen ist, kann die Platte 110 jeweils über eine Schlüsselloch-Verbindung 114 an den oberen Enden der Säulen 84,88 festgelegt sein.

Zur Halterung des Stapels 12 in Arbeitshöhe vor der Höhenverstelleinrichtung 56 können die Säulen 84,88 abgestuft abgebildet sein mit durchmesservergrößerten unteren Abschnitten 115,116. Auf der Stufen-Kreisringfläche am oberen Enden der Abschnitte 115,116 ruht eine Auflageplatte 118, auf die wiederum der Plattenstapel 12 aufgesetzt ist.

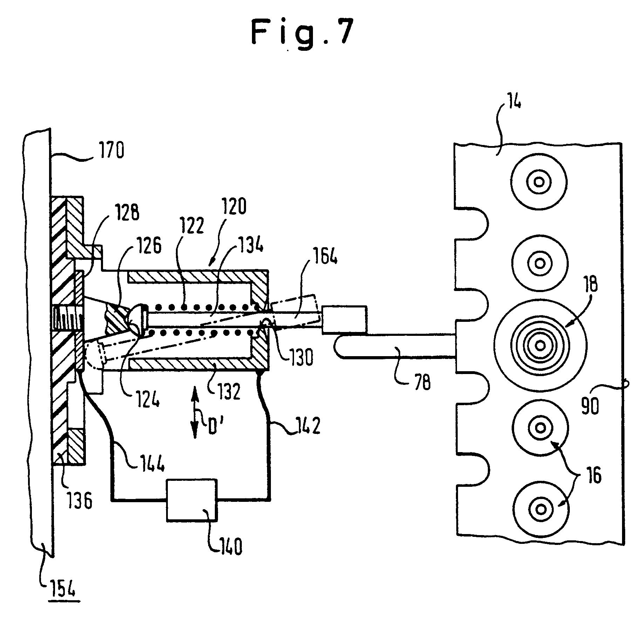

Bei gelöster Spanneinrichtung (Doppelarmhebel 80) können die Platten 14 in der beschriebenen Weise ohne weiteres seitlich verschoben werden, da das Gewicht des Plattenstapels bei der dargestellten Anzahl noch nicht allzu hoch ist (Platten 14, insbesondere aus Kunststoff). Die geringfügig über die jeweilige Plattenoberseite 24 im entspannten Zustand vorstehenden O-Ringe 26,34 verhindern ein "Festsaugen" der Platten aneinander. In Extremfällen oder auch bei erhöhter Plattenanzahl kann es u.U. dennoch zu einem Blockieren einer gerade zu verstellenden Platte kommen. Um in diesem Falle eine Beschädigung der Vorrichtung auszuschließen, kann gemäß Fig. 7 eine Überlastsicherung 120 vorgesehen sein. Diese wird vom elektrisch leitfähig ausgebildeten, stiftförmigen Plattenverschiebeteil 164 gebildet, der von einer Spiral-Vorspannfeder 122 gegen einen konkaven Sitz 124 eines Isolierteils 126 gedrückt wird. Das Isolierteil 126 ist am Kopf 154 starr befestigt. Das Isolierteil 126 steht von einer kreisscheibenförmigen Kontaktplatte 128 ab. Der Schaft des stiftförmigen Plattenverschiebeteils 126 zwischen beiden verdickten Enden ist in einer Führungsöffnung 130 eines vom Kopf 154 abstehenden Gehäuses 132 verschiebbar und in gewissem Grade verkippbar geführt, wobei zum einen über die Randberührung des Schafts 134 in der Führungsöffnung 130 und zum anderen über die sich sowohl am elektrisch leitenden Gehäuse 132 rings um die Öffnung 130 als auch am inneren verdickten Ende des Teils 164 abstützende Feder 122 eine elektrisch leitende Verbindung zwischen dem Schaft 134 und dem Gehäuse 132 besteht. Das Gehäuse 132 ist vom Kopf 154 über eine Isolierplatte 136 elektrisch isoliert. Die Kontaktplatte 128 ist innerhalb des Gehäuses 132 mit Abstand zu diesem an der Isolierplatte 136 befestigt und demzufolge vom Gehäuse 132 elektrisch isoliert. Wird nun auf das freie Ende des Plattenverschiebeteils 164 bei der Verschiebung des das Gehäuse 132 tragenden Teils 170 (entsprechend dem Teil 70 in Fig. 2) in Richtung D' ein entsprechend großer Drehmoment ausgeübt, so gleitet das innere Ende des Plattenverschiebeteils 164 vom Sitz 124 und wird anschließend von der Feder 122 gege die Kontaktplatte 128 gedrückt. Sind nun die Kontaktplatte 128 und das Gehäuse 132 Teile eines Stromkreises, so wird dieser bislang offene Stromkreis nunmehr durch das Plattenverschiebeteil 122 geschlossen. Dies kann zum Abschalten des Motors 62 eingesetzt werden. In Fig. 7 erkennt man eine als Block 140 dargestellte Steuerung für den Motor 62, der über eine Anschlußleitung 142 mit dem Gehäuse 132 über eine Anschlußleitung 144 mit der Kontaktplatte 128 leitend verbunden ist. Die Steuerung sorgt im angesprochenen Überlast-Fall für ein Abschalten des Motors 62.

Aufgrund der roationssymmetrischen Anordnung von Kontaktplatte 122, konischem Isolierteil 126, Plattenverschiebeteil 122 und Gehäuse 132 ergibt sich auch dann ein Erschließen des Stromkreises, wenn das Plattenverschiebeteil 164 in anderer Richtung überlastet wird, beispielsweise dann, wenn bei einer Vertikalbewegung des Kopfes 40 das Plattenverschiebeteil 164 gegen irgendein Hindernis, beispielsweise einen der stiftförmigen Gegenvorsprünge 78, stößt. Die Steuerung 140 schaltet folglich beide Motoren 60 und 62 ab.

## Patentansprüche

1. Vorrichtung zur Durchführung chemischer Reaktionsfolgen mit
   – einem Stapel (12) übereinander angeordneter und wahlweise gegeneinander in zur Stapelrichtung senkrechter Verschieberichtung schrittweise verschiebbarer Reaktionsplatten (14), die mit im Schrittabstand (a) angeordneten Durchgängen (16) versehen sind, von denen jeweils eine als Reaktionskammer (18) ausgebildet ist,
   – einer Plattenverstelleinrichtung (56) zur wahlweisen Verschiebung der jeweiligen Platte (14) relativ zum übrigen Plattenstapel (12) mittels eines wahlweise antreibbaren Plattenverschiebeteils (64), welches an einem in Stapelrichtung zwischen den einzelnen Platten (14) zugeordneten Plattenverstellpositionen wahlweise bewegbaren Kopf (54), der Plattenverstelleinrichtung (56) beweglich gelagert ist, und
   – einer Spanneinrichtung mit einem an den Plattenstapel (12) angreifenden Andrückteil (100), welches zwischen einer Andrückstellung mit abdichtendem Zusammenspannen der Platten (16) und einer Freigabestellung mit Bewegungsmöglichkeit der Platten (14) gegeneinander bewegbar ist,
dadurch gekennzeichnet, daß die Spanneinrichtung eine mit dem Andrückteil (100) verkoppelte Kraftübertragungseinrichtung umfaßt, an welche die Plattenverstelleinrichtung angreift zur Bewegung des Andrückteils aus der Freigabestellung in die Andrückstellung bei einer Bewegung des Kopfes (54) der Plattenverstelleinrichtung (56) über eine der beiden äußeren Plattenverstellpositionen hinaus in eine Endposition.

2. Vorrichtung nach Anspruch 1, dadurch gekennzeichnet, daß die Kraftübertragungseinrichtung einen Doppelarmhebel (80) umfaßt, dessen eines Ende mit der Plattenverstelleinrichtung und dessen anderes Ende mit dem Andrückteil (100) zusammenwirkt.

3. Vorrichtung nach Anspruch 2, dadurch gekennzeichnet, daß wenigstens eines der Enden des Doppelarmhebels (80) mit einer Spanndruck-Einstelleinrichtung versehen ist.

4. Vorrichtung nach Anspruch 3, dadurch gekennzeichnet, daß die Spanndruck-Einstelleinrichtung von einem verstellbaren Doppelkeil (94) gebildet ist.

5. Vorrichtung nach wenigstens einem der Ansprüche 2 - 4, dadurch gekennzeichnet, daß der Kopf (54) der Plattenstelleinrichtung (56) an seiner Oberseite mit einem Vorsprung (97) versehen ist, welcher bei einer Bewegung des Kopfes (54) über die oberste Plattenverstellposition hinaus in die Endposition an das eine Ende des Doppelarmhebels (80) angreift.

6. Vorrichtung nach wenigstens einem der vorhergehenden Ansprüche mit einer mit der Plattenverstelleinrichtung (56) bewegungsverkoppelten Verschiebesperre für die jeweilige Platte unmittelbar oberhalb der von der Plattenverstelleinrichtung jeweils angefahrenen Platte, dadurch gekennzeichnet, daß die Verschiebesperre am Kopf (54) gehaltert ist.

7. Vorrichtung nach Anspruch 6, dadurch gekennzeichnet, daß die Verschiebesperre auch an die unmittelbar unterhalb der von der Plattenverstelleinrichtung (56) angefahrenen Platte (14) angeordnete Platte (14) sperrend angreift.

8. Vorrichtung nach Anspruch 6 oder 7, dadurch gekennzeichnet, daß am Kopf (54) oberhalb und unterhalb des Plattenverschiebeteils (64) je ein Eingriffselement (52) starr angeordnet ist, welches mit an den Platten (14) angeordneten Gegeneingriffselementen zusammenwirkt.

9. Vorrichtung nach Anspruch 8, dadurch gekennzeichnet, daß eines der Elemente Eingriffselement - Gegeneingriffselement einen Eingriffsvorsprung (50,52) umfaßt, und daß das andere Element mehrere, in Verschieberichtung nebeneinander im Schrittabstand angeordnete, zur Stapelrichtung parallel verlaufende, im Querschnitt an den Eingriffsvorsprung angepaßte Eingriffs-Nuten (72) umfaßt.

10. Vorrichtung nach Anspruch 8, dadurch gekennzeichnet, daß eines der Elemente Eingriffselement - Gegeneingriffselement eine zur Stapelrichtung parallel verlaufende Eingriffs-Nut umfaßt, und daß das andere Element mehrere, in Verschieberichtung nebeneinander im Schrittabstand angeordnete, an den Nut-Querschnitt angepaßte Eingriffsvorsprünge umfaßt.

11. Vorrichtung nach Anspruch 9, dadurch gekennzeichnet, daß das Andrückteil (100) mit einer Eingriffsnut (98) für den oberen Eingriffsvorsprung (52) des Kopfes (54) ausgebildet ist.

12. Vorrichtung nach wenigstens einem der vorhergehenden Ansprüche, dadurch gekennzeichnet, daß das Plattenverschiebeteil mit einem in Verschieberichtung beweglichen Verschiebevorsprung (76) ausgebildet ist, welcher mit an den Platten (14) ausgebildeten Gegenvorsprüngen (78) zusammenwirkt.

13. Vorrichtung nach Anspruch 12, **dadurch gekennzeichnet**, daß der Verschiebevorsprung (76) in einer Zwischenposition des Kopfes (54) zwischen aufeinanderfolgenden Plattenverstellpositionen frei zwischen den Gegenvorsprüngen (78) aufeinanderfolgender Platten (14) hindurch bewegbar ist.

14. Vorrichtung nach Anspruch 12 oder 13, **dadurch gekennzeichnet**, daß der Verschiebevorsprung (76) und/oder die Gegenvorsprünge (78) stiftförmig sind.

15. Vorrichtung nach wenigstens einem der Ansprüche 12 - 14, **dadurch gekennzeichnet**, daß das Plattenverschiebeteil (164) mit einer Überlastsicherung zusammenwirkt.

16. Vorrichtung nach Anspruch 15, **dadurch gekennzeichnet**, daß das stiftförmige Plattenverschiebeteil (164) elektrisch leitfähig ausgebildet ist, daß eine Vorspannfeder (122) in einer Normalstellung des Plattenverschiebeteils (164) dieses gegen ein Isolierteil (126) andrückt, und daß bei Überlast das Plattenverschiebeteil (164) vom Isolierteil (126) abgeleitet und von der Vorspannfeder (122) gegen eine elektrisch leitende Kontaktplatte (128) gedrückt wird.

17. Vorrichtung nach wenigstens einem der vorhergehenden Ansprüche, **gekennzeichnet durch** O-Ring-Dichtungen (26,34) zwischen aufeinanderfolgenden Platten (14) zur Abdichtung der Durchgänge (16) nach außen hin.

18. Vorrichtung nach wenigstens einem der vorhergehenden Ansprüche, dadurch gekennzeichnet, daß den Durchgängen (16) des Stapels (12) Flüssigkeit über eine Misch-Ventilanordnung (51) zugeführt wird.

## Claims

1. Apparatus for carrying out chemical reaction sequences, having
   – a stack (12) of reaction plates (14) arranged one above another and optionally displaceably in relation to one another step by step in a direction of displacement perpendicular to the stacking direction, which plates are provided with passages (16) arranged at the stepping interval (a), one of which in each case is formed as reaction chamber (18),
   – a plate displacement device (56) for the optional displacement of the respective plate (14) in relation to the remaining plate stack (12) by means of a plate displacement part (64) drivable according to choice, which is movably mounted on a head (54) of the plate displacement device (56) which is movable according to choice in the stack direction between plate displacement positions allocated to the individual plates (14), and
   – a tightening device having a presser part (100) acting on the plate stack (12), which presser part is movable between a pressure application position with sealing clamping together of the plates (16) and a release position with possibility of movement of the plates (14) in relation to one another
characterised in that the clamping device comprises a forcetransmission device coupled with the presser part (100), on which device the plate displacement device acts for the movement of the presser part out of the release position into the pressure application position on a movement of the head (54) of the plate displacement device (56) beyond one of the two outer plate displacement positions into an end position.

2. Apparatus according to Claim 1, characterised in that the force transmission device comprises a two-armed lever (80) of which one end co-operates with the plate displacement device and the other end co-operates with the pressure application part (100).

3. Apparatus according to Claim 2, characterised in that at least one of the ends of the two-armed lever (80) is provided with a clamping pressure adjusting device.

4. Apparatus according to Claim 3, characterised in that the clamping pressure adjusting device is formed by an adjustable double wedge (94).

5. Apparatus according to at least one of Claims 2 - 4, characterised in that the head (54) of the plate displacement device (56) is provided on its upper side with a projection (97) which, on a movement of the head (54) beyond the uppermost plate displacement position into the end position, acts on the one end of the two-armed lever (80).

6. Apparatus according to at least one of the preceding Claims, having a shift lock, coupled in movement with the plate displacement device (56), for the respective plate directly above the plate approached in each case by the plate displacement device, characterised in that the shift lock is retained on the head (54).

7. Apparatus according to Claim 6, characterised in that the shift lock also acts in blocking manner upon the plate (14) arranged immediately beneath the plate (14) approached by the plate displacement device (56).

8. Apparatus according to Claim 6 or 7, characterised in that an engagement element (52) is arranged rigidly on the head (54), both above and below the plate displacement part (64), which element co-operates with counter-engagement elements arranged on the plates (14).

9. Apparatus according to Claim 8, characterised in that one of the elements, engagement element or counter-engagement element, comprises an engagement projection (50, 52), and in that the other element comprises a plurality of engagement grooves (72) arranged side by side in the shift direction at the stepping interval, extending parallel to the stacking direction and adapted in cross-section to the engagement projection.

10. Apparatus according to Claim 8, characterised in that one of the elements, engagement element and counter-engagement element, comprises an engagement groove extending parallel with the stacking direction, and in that the other element comprises a plurality of engagement projections arranged at the step interval side by side in the displacement direction and adapted to the groove cross-section.

11. Apparatus according to Claim 9, characterised in that the pressure application part (100) is formed with an engagement groove (98) for the upper engagement projection (52) of the head (54).

12. Apparatus according to at least one of the preceding Claims, characterised in that the plate displacement part is formed with a displacement projection (76) movable in the displacement direction, which co-operates with counter-projections (78) formed on the plates (14).

13. Apparatus according to Claim 12, characterised in that the displacement projection (76), in an intermediate position of the head (54) between successive plate displacement positions, is movable freely through between the counter-projections (78) of successive plates (14).

14. Apparatus according to Claim 12 or 13, characterised in that the displacement projection (76) and/or the counter-projections (78) are of pin form.

15. Apparatus according to at least one of Claims 12 - 14, characterised in that the plate displacement part (164) co-operates with an overload safety device.

16. Apparatus according to Claim 15, characterised in that the plate displacement part (164) of pin form is made electrically conductive, in that an initial stress spring (122), in a normal position of the plate displacement part (164), presses the latter against an insulating part (126), and in that in the case of overloading the plate displacement part (164) slides off from the insulating part (126) and is pressed by the initial stress spring (122) against an electrically conductive contact plate (128).

17. Apparatus according to at least one of the preceding Claims, characterised by O-ring seals (26, 34) between successive plates (14) for the sealing of the passages (16) to the exterior.

18. Apparatus according to at least one of the preceding Claims, characterised in that liquid is fed to the passages (16) of the stack (12) by way of a mixer valve arrangement (51).

## Revendications

1. Dispositif pour effectuer une séquence de réactions chimiques, avec :
    – un empilement (12) de plaques de réaction (14), superposées et déplaçables au choix les unes par rapport aux autres, pas à pas, dans une direction de déplacement perpendiculaire à la direction de l'empilement, plaques de réaction (14) pourvues de passages (16) disposés selon un espacement de pas (a), l'un des passages étant chaque fois réalisé sous forme de chambre de réaction (18),
    – un dispositif de déplacement de réglage des plaques (56), pour déplacer au choix la plaque (14) concernée, par rapport au reste de l'empilement de plaques (12), au moyen d'une partie de déplacement de plaque (61) susceptible de subir un entraînement au choix, qui est montée sur une tête (54), susceptible d'être déplacée à volonté entre les différentes positions de réglage de plaque associées aux différentes plaques, du dispositif de réglage de plaques (56), et
    – un dispositif de serrage, avec une partie de pressage (100) agissant sur l'empilement de plaques (12), qui est déplaçable entre une position d'application de pression, où les plaques (16) sont comprimées les unes les autres de façon étanche, et une position de libération, offrant une possibilité de déplacement aux plaques (14),
caractérisé en ce que le dispositif de serrage comprend un dispositif de transmission de force, couplé à la partie de serrage (100), sur lequel agit le dispositif de réglage de plaques, en vue de déplacer la partie de pressage hors de la position de libération, en position d'application de pression, lors d'un déplacement de la tête (54) du dispositif de réglage de plaques (56), en passant sur l'une des deux positions extérieures de réglage de plaques, en arrivant en une position finale.

2. Dispositif selon la revendication 1, caractérisé en ce que le dispositif de transmission de forces comprend un levier à deux bras, dont une extrémité coopère avec le dispositif de réglage de plaques et l'autre extrémité coopère avec la partie de pressage (100).

3. Dispositif selon la revendication 2, caractérisé en ce qu'au moins l'une des extrémités du levier à deux bras (80) est pourvue d'un dispositif de réglage de la pression de serrage.

4. Dispositif selon la revendication 3, caractérisé en ce que le dispositif de réglage de la pression de serrage est formé par un coin double (94) réglable.

5. Dispositif selon l'une au moins des revendications 2 à 4, caractérisé en ce que la tête du dispositif de réglage de plaques (56) est pourvue en face supérieure d'une saillie (97) qui, lors d'un déplacement de la tête (54), agit sur la position la plus haute de réglage des plaques, dans la position finale, sur une extrémité du levier à deux bras (80).

6. Dispositif selon l'une au moins des revendications précédentes, avec un verrou de déplacement, couplé en déplacement au dispositif de réglage des plaques (56), affecté à la plaque qui est directement au-dessus de celle qui vient d'être déplacée par le dispositif de réglage de plaques, caractérisé en ce que le verrou de déplacement est fixé sur la tête (54).

7. Dispositif selon la revendication 6, caractérisé en ce que le verrou de déplacement agit également pour bloquer la plaque (14) disposée directement au-dessous de la plaque (14) déplacée par le dispositif de réglage de plaques (56).

8. Dispositif selon la revendication 6 ou 7, caractérisé en ce qu'un élément de prise (52), qui coopère avec des contre-éléments de prise disposés sur les plaques (14), est chaque fois disposé fixement sur la tête (54), respectivement au-dessus et au-dessous de la partie de déplacement de plaque ( 64 ).

9. Dispositif selon la revendication 8, caractérisé en ce que l'un des éléments que sont l'élément de prise - le contre-éléments de prise, comprend une saillie d'engagement (50,52), et que l'autre élément comprend plusieurs rainures d'engagement (72), disposées selon un espacement, les unes à côté des autres dans la direction du déplacement, en s'étendant parallèlement à la direction de l'empilement, rainures dont la section transversale est adaptée à la saillie d'engagement.

10. Dispositif selon la revendication 8, caractérisé en ce que l'un des éléments que sont l'élément de prise - le contre-éléments de prise, comprend une rainure d'engagement, qui s'étend parallèlement à la direction de l'empilement, et que que l'autre élément comprend plusieurs saillies d'engagement, disposées selon un espacement, les unes à côté des autres dans la direction du déplacement, saillies dont la section transversale est adaptée à la rainure d'engagement.

11. Dispositif selon la revendication 9, caractérisé en ce que la partie de pressage (100) est réalisée avec une rainure d'engagement (98) pour la saillie d'engagement supérieure (52) de la tête (54).

12. Dispositif selon l'une au moins des revendications précédentes, caractérisé en ce que la partie de déplacement de plaques est réalisée avec une saillie de déplacement (76), mobile dans la direction du déplacement, qui coopère avec des contre-saillies (78) réalisées sur les plaques (14).

13. Dispositif selon la revendication 12, caractérisé en ce que, dans une position intermédiaire de la tête (54), la saillie de déplacement (76) est déplaçable librement entre les contre-saillies (78) de plaques (14) qui se suivent, entre des positions successives de réglage de plaques.

14. Dispositif selon la revendication 12 ou 13, caractérisé en ce que la saillie de déplacement (76) et/ou les contre-saillies (78) ont la forme de tiges.

15. Dispositif selon l'une au moins des revendications 12 à 14, caractérisé en ce que la partie de déplacement de plaques (164) coopère avec une sécurité de surcharge.

16. Dispositif selon la revendications 15, caractérisé en ce que la partie de déplacement de plaques (164) en forme de tige est réalisée de façon à pouvoir conduire l'électricité, qu'en une position normale de la partie de déplacement de plaques (164), un ressort de précontrainte (12) presse celle dernière contre une partie isolante (126), en que, en cas de surcharge, la partie de déplacement de plaques (164) est isolée par la partie isolante (126) et est pressée, par le ressort de précontrainte (122), contre une plaque de contact (128) conductrice de l'électricité.

17. Dispositif selon l'une au moins des revendications précédentes, caractérisé par des étanchéités à joints toriques (26,34), entre les plaques (14) superposées, en vue d'isoler hermétiquement les passages (16) vers l'extérieur.

18. Dispositif selon l'une au moins des revendications précédentes, caractérisé en ce qu'un liquide est amené aux passages (16) de l'empilement (12), par un dispositif de mélange à valve (51).

# Fig.1

Fig.2

# Fig. 3

Fig. 4

Fig. 5

Fig. 6

# Fig.7